# EUROPEAN PATENT APPLICATION

(11) **EP 3 391 879 A1**
(43) Date of publication of application: **24.10.2018**
(21) Application number: 16876104.7
(22) Date of filing: 23.05.2016
(51) Int. Cl.: A61K 31/045, A61K 31/11, A61K 31/165, A61K 31/20, A61K 31/215, A61K 31/23, A61K 31/405, A61K 31/52, A61K 31/60, A61K 9/06, A61K 9/10, A61P 17/02

(54) **PHARMACEUTICAL COMPOSITION FOR TREATING SKIN ULCERS, INJURIES AND BURNS**

(30) Priority: 16.12.2015 MX 2015017409
(71) Applicant: Guerrero Gonzalez, Tayde, Ciudad de México 11360 (MX)
(72) Inventor: Guerrero Gonzalez, Tayde, Ciudad de México 11360 (MX)
(74) Representative: Cohausz & Florack
(86) International application number: PCT/MX2016/000050
(87) International publication number: WO 2017/105207

(57) **Abstract**

The present invention relates to a topical pharmaceutical composition which can be used to treat skin ulcers, injuries and burns, and which contains, in addition to a pharmaceutically acceptable excipient, acetic indole acid, butyric indole acid, salicylic acid, acetyl benzamide, dibenzene carboxylic acid, decanoic acid, dodecanoic acid, tetradecanoic acid, hexadecanoic acid, 9-octadecanoic acid, octadecanoic acid methyl ester, 1-decanol, nonanal, 13-octadecenal, kinetin, gibberellic acid, and gibberellins.

## Description

### TECHNICAL FIELD

The present disclosure relates to pharmaceutical compositions useful for the treatment of wounds, burns and ulcers; more particularly, relates to pharmaceutical compositions useful for the treatment of diabetic foot ulcers.

### BACKGROUND OF THE INVENTION

Diabetes mellitus (DM) is one of the main problems in health systems and a global public health threat that has dramatically increased in the last two decades. According to epidemiological studies, the number of patients with DM increased from about 30 million cases in 1985, 177 million in 2000, 285 million in 2010, and estimates that if the situation continues, more than 360 million people by the year 2030 will have DM .

To date, diabetic foot ulcer (UPD) is considered as a major source of morbidity and a major cause of hospitalization in patients with diabetes. It is estimated that approximately 20% of hospital admissions in patients with DM are the result of UPD. In fact, the UPD can lead to infections, gangrene, amputation and even death if necessary care is not provided. On the other hand, once the UPD has been developed, there is a greater risk of ulcer progression that can ultimately lead to amputation. In general, the rate of amputation of lower limbs in patients with DM is 15 times higher than in patients without diabetes. It is estimated that approximately 0-70% of all lower limb amputations are due to PD. In addition, it has been reported that worldwide every 30 seconds a leg is amputated due to UPD. In addition, the UPD is responsible for emotional distress and considerable physical disability of the patient, which affects their productivity and generates financial losses that impact their quality of life.

Studies indicate that the cure of a single ulcer costs approximately US $2000. In cases where amputation of lower limbs is required, health care is even more expensive at around 3500-4000 dolars. These costs do not represent the total economic burden to which the indirect costs related to the loss of productivity, the prevention, rehabilitation and home care efforts must be considered. When all this is considered, 7% and 20% of the total number of cases of diabetes in North America and Europe, respectively, could be attributed to UPD.

In the treatment of skin wounds, particularly those with diabetic foot, various modalities have been used. For example, patent application EP1970071 describes the use of topical formulations containing epidermal growth factor ncapsulated or associated with deformable or conventional liposomes to be applied on the surface of and around skin lesions.

Likewise, the patent application CN104800265 describes an oad useful for the treatment of diabetic foot wounds, which comprises metronidazole, notoginseng powder, safflower oil, and vitamine B1. This ointment promotes blood circulation, as well as elimination of necrotic tissue, the promotion of granulation and the reduction of inflammation.

On the other hand, the patent application US2014377249 describes ompositions containing hyaluronic acid or derivatives thereof in association with the enzyme collagenase, which are placed in a bandage for the topical treatment of various wounds, burns, or ulcers, including of diabetic foot.

Similarly, patent application US2014066402 discloses an omposition comprising glycosaminoglycans, specifically low molecular weight heparins (LMWHs) and very low molecular weight (VLMWHs). Said composition is useful for the treatment of chronic ulcers, especially those diabetic foot ulcers.

Finally, patent application US2012196930 describes a topical pharmaceutical gel composition useful for the treatment of hurts, which comprises methyl-3-[4-(2-hydroxy-3-isopropyl-amino)-propoxy]phenylpropionate chloride, Pluracare® F108NF, benzalconium, sodium acetate trihydrate, glacial acetic acid, and hydrochloric acid.

### BRIEF SUMMARY OF THE INVENTION

In a first aspect, the present invention provides a topical pharmaceutical composition containing as active principle the following components: a) indole acetic acid, b) indole butyric acid, c) salicylic acid, d) acetyl benzamide, e) dibenzene carboxilyc acid, f) decanoic acid. g) dodecanoic acid, h) tetradecanoic acid, i) hexadecanoic acid, j) 9-octadecanoic acid, k) octadecanoic acid methyl ester, I) 1-decanol, m) nonanal, n) 13-octadecenal, o) kinetin, p) gibberelic acid, p) gibberellins.

Another preferred aspect of the present invention relates to a pharmaceutical composition prepared in solid form or aqueous suspension, in a pharmaceutically acceptable diluent. Pharmaceutical composition provided by the present invention can be administered by any available route of administration, for which said composition is formulated in a pharmaceutical form adapted to the route of administration. In a preferred aspect, the medically is adapted for a topical, dermal, cutaneous, subcutaneous form. In the case of a wound, the administration can be performed directly on it.

### DETAILED DESCRIPTION

The present invention features various topical pharmaceutical formulations useful for the treatment of wounds, burns, and waxes, particularly of ulcers in diabetic foot, which contains as active principle the following components: a) indole acetic acid, b) indole butyric acid, c) salicylic acid, d) acetyl benzamide, e) dibenzene carboxilyc acid, f) decanoic acid. g) dodecanoic acid, h) tetradecanoic acid, i) hexadecanoic acid, j) 9-octadecanoic acid, k) octadecanoic acid methyl ester, I) 1-decanol, m) nonanal, n) 13-octadecenal, o) kinetin, p) gibberelic acid, p) gibberellins.

Active principle may be in the range of about 0.001% to about 50%. More preferred concentrations of the active principle are in the range of around 1% to around 20%. Most preferred concentrations of the active ingredient are in the range of about 10% to about 0%.

Indole acetic acid component of the active ingredient may be in the range of about 0.001% to about 50%. More preferred indole acetic acid concentrations are in the range of about 1% to about 20%. Most preferred concentrations of indoic acid are in the range of about 10% to about 20%.

Indole butyric acid component of the active ingredient may be in the range of about 0.001% to about 50%. More preferred indole acid concentrations are in the range of about 1% to about 20%. Most preferred concentrations of indole butyric acid are in the range of about 10% to about 20%.

Salicylic acid component of the active ingredient may be in the range of about 0.001% to about 0%. More preferred concentrations of salicylic acid are in the range of about 1% to about 20%. Most preferred concentrations of salicylic acid are in the range of about 10% to about 20%.

Acetyl benzamide component of the active ingredient may be in the range of about 0.001% to about 0%. More preferred concentrations of acetyl benzamide are in the range of about 1% to about 20%. Most preferred concentrations of acetyl benzamide are in the range of about 10% to about 20%.

Dibenzene carboxylic acid component of the active ingredient may be in the range of about 0.001% to about 50%. More preferred concentrations of the ibenocene carboxylic acid are in the range of about 1% to about 20%. Most preferred concentrations of dicarboxylic acid are in the range of about 10% to about 20%.

Decanoic acid component of the active ingredient may be in the range of about 0.001% to about 0%. More preferred concentrations of decanoic acid are in the ango from about 1% to about 20%. Most preferred concentrations of decanoic acid are in the range of about 10% to about 20%.

Dodecanoic acid component of the active ingredient may be in the range of about 0.001% to about 50%. More preferred concentrations of dodecanoic acid are in the range of about 1% to about 20%. Most preferred concentrations of dodecanoic acid are in the range of about 10% to about 20%.

Tetradecanoic acid component of the active ingredient may be in the range of about 0.001% to about 50%. Mosre preferred concentrations of tetradecanoic acid are in the range of about 1% to about 20%. Most preferred concentrations of tetradecanoic acid are in the range of about 10% to about 20%.

Hexadecanoic acid component of the active ingredient may be in the range of about 0.001% to about 50%. More preferred concentrations of hexadecanoic acid are in the range of about 1% to about 20%. Most preferred concentrations of hexadecanoic acid are in the range of about 10% to about 20%.

9-octadecanoic acid component of the active ingredient may be in the range of about 0.001% to about 50%. More preferred concentrations of 9-octadecanoic acid are in the range of about 1% to about 20%. Most preferred concentrations of 9-octadecanoic acid are in the range of about 10% to about 20%.

Octadecanoic acid methyl ester component of the active ingredient may be in the range of about 0.001% to about 50%. More preferred concentrations of octadecanoic methyl ester are in the range of about 1% to about 20%. Most preferred concentrations of the octadecanoic acid methyl ester are in the range of about 10% to about 20%.

1-decanol of the active ingredient may be in the range of about 0.001% to about 50%. More preferred concentrations of 1-decanol are in the range of around 1% to around 20%. Most preferred concentrations of 1-decanol are in the range of about 10% to about 20%.

Nonanal component of the active ingredient may be in the range of about 0.001% to about 50%. More preferred concentrations of Nonanal are in the range of around 1% to around 20%. Most preferred concentrations of nonanal are in the range of about 10% to about 20%.

13-octadecenal component of the active ingredient of the present invention may be in the range of about 0.001% to about 50%. More preferred concentrations of 13-octadecenal are in the range of about 1% to about 20%. Most preferred 13-octadecenal concentrations are in the range of about 10% to about 20%.

Kinetin component of the active ingredient of the present invention may be in the range of about 0.001% to about 50%. More preferred concentrations of kinetin are in the range of about 1% to about 20%. Most preferred concentrations of kinetin are in the range of about 10% to about 20%.

Gibberellic acid component of the active ingredient of the present invention may be in the range of about 0.001% to about 50%. More preferred concentrations of gibberellic acid are in the range of about 1% to about 20%. Most preferred concentrations of gibberellic acid are in the range of about 10% to about 20%.

Gibberellin component of the active ingredient of the present invention may be in the range of about 0.001% to about 50%. More preferred concentrations of gibberellins are in the range of about 1% to about 20%. Most preferred concentrations of gibberellins are in the range of about 10% to about 20%.

The term "pharmaceutical composition" refers to any substance used for the prevention, diagnosis, alleviation, treatment or cure of diseases in humans or animals. The pharmaceutical composition of the present invention can be used alone or in combination with other pharmaceutical compositions.

Pharmaceutical composition further comprises a pharmaceutically acceptable excipient and/or carrier, and/or an adjuvant, and/or other active ingredient, or any combination thereof. As used herein, the term "active ingredient" ("active substance", "pharmaceutically active substance", "active ingredient", or "pharmaceutically active ingredient") means any component that provides a potentially pharmaceutical activity or a different effect on the cure, mitigation, treatment, or prevention of disease, or affect the structure or function of the human body or animals.

The dosage regimen of the pharmaceutical composition of the present invention for an adult can be estimated by interpolations widely known in the state of the art.

A pharmaceutical form is an individualized provision to which the medicinal substances (active ingredients) and excipients are adapted to constitute a medicament; that is, the way to prepare a medication for the purpose of its administration. This pharmaceutical form is the product resulting from the technological process that gives the drugs suitable characteristics, such as: dosage, therapeutic efficacy and stability over time.

We can distinguish pharmaceutical forms of conventional release, where the release of the active principle is not deliberately modified by a particular formulation design; and the pharmaceutical forms of modified release, which allow reaching a plasma concentration profile that guarantees the persistence of the therapeutic action of the drug.

The pharmaceutical forms are classified according to their physical state: a) solid (powders, granules, capsules, tablets, tablets, suppositories, ovules, and implants), b) liquid (solutions, suspensions, emulsions, syrups, elixirs, lotions, liniments and injectables, and c) semi-solid (ointments, pastes, creams and gels).

The semi-solid pharmaceutical forms are intended to be applied to the skin or to certain mucous membranes in order to exert a local action or to give rise to the cutaneous penetration of the medicaments they contain. These consist of an excipient, simple or complex, in which the active ingredients are dissolved or dispersed. Generally, these forms are classified into the following groups: a) cream, ointments, and pastes, and b) gels.

Pharmaceutical formulations in cream presentation. Pharmaceutical formulations in cream presentation are in two phases, a lipophilic and another aqueous or hydrophilic. These may be liquid or semi-solid preparations containing the active ingredient (s) and additive (s) necessary to obtain an emulsion, generally oil in water, with a water content greater than 20%. This type of formulations are used massively for skin care, analgesics, anti-inflammatories, healing of cutaneous wounds, etc. The composition of the creams consists of an aqueous phase, an oil phase, and an emulsifier system.

The most common errors in the manufacture of creams include: a) oil phase disintegrated due to the use of an insufficient amount of emulsifier, b) formation of lumps due to temperature difference between the two phases, c) separation between the two phases due to a rapidity of mixing between them, d) air bubbles due to improper and irregular agitation, e) separation of phases due to lack of agitation before cooling the cream, and f) alteration of components due to heating at very high temperature.

The characteristics of a cream include: a) good tolerance (no irritation, or sensitization), b) inertia against the active ingredient and conditioning material, c) stability against ambient factors, d) convenient consistency for easy spreading on the skin and dispensing in tubes, e) pleasant organoleptic characteristics, f) ability to incorporate substances soluble in water and in oil, g) ability to act on oily or dry skin, h) ease to quickly transfer active substances to the skin, i) Do not dehydrate or degrease the skin.

For topical treatment there are two main components, active principle and excipient. Secondary products are also included, such as preservatives, flavorings and colorants. The active principle (re-epithelialization-inducing composition) is determined by the disease to be treated. On the other hand, the excipient has the function of transporting the active principle towards the interior of the skin. Both are equally important, since the use of an appropriate active ingredient in a wrong excipient can cause a dermatosis.

An active ingredient in a liquid vehicle is poorly absorbed because it evaporates easily and on the other hand the stratum corneum is hydrophobic. If we use a fatty vehicle such as ointments or ointments, the absorption is maximum since the excipient accumulates in the stratum corneum and slowly releases the drug into the skin, thanks to the concentration gradient.

The creams are classified as hydrophobic and hydrophytic. In hydrophobic creams the continuous or external phase is the lipophilic phase due to the presence in its composition of W/O type emulsifiers. In cases of dry piet or chronic dermatosis, e! use of emulsions of this type. The internal phase consists of water droplets surrounded by the oil phase, they are not absorbed so quickly in the skin, they have an occlusive effect that reduces the transepidermal water loss in the skin. They are suitable for releasing active ingredients in the skin and can not be washed with water alone.

For its part, in the hydrophytic creams the external phase is of aqueous nature due to the presence in its composition of O/W type emulsifiers, such as sodium or triethanolamine soaps, sulfated fatty alcohols and polysorbates sometimes combined in convenient proportions with type W/O emulsifiers. In cases of normal skin or presence of slight dryness, the use of a O/W emulsion is recommended since the oil droplets of the preparation are placed inside the aqueous phase, they are quickly absorbed in the skin without leaving an oily trace, the aqueous part evaporates generating a cooling effect, the oil phase greases the skin.

The excipients are auxiliary substances that help to formulate a specific pharmaceutical form in a better way. The main function of the excipient is to support the active principle that is desired to be applied to the skin, although the excipient may influence the penetration of the active principle towards less or deeper places located below the application area and thus contribute to effectiveness of drug. In other cases, the excipient contributes to maintain the physicochemical characteristics of normal skin (moisture and pH), thus improving their defense mechanisms.

The characteristics that the excipients of creams must meet are the following:
a) well tolerated and not to manifest actions that disable them (irritants, sensitizers),
b) inert against the active principle that they incorporate (physical and chemical compatibility) as well as against the material conditioning, c) stability against environmental factors to ensure their preservation, d) suitable consistency so that its extension on the skin or mucous cavities is easily performed and dispersed in tubes, e) sterilizable in some cases (ophthalmic ointments), f) yield adequately the active principle that they incorporate; g) lack, as far as possible, unpleasant organoleptic characteristics.

Stearic acid. This acid is used as an emulsifier for the formation of base creams, sometimes used as evanescent emulsions, partially neutralized with an alkali (mainly triethanolamine). The free stearic acid in these creams produces a pearly appearance. Creams of stearic acid may appear cracked by drying or with lumps, due to reactions of this with zinc or calcium salts. It is used analogously to white wax for ointments and ceratos. It helps the stability of W/O emulsions (aqueous/oily), has emollient and protective properties and is easily absorbed by the skin. It is also used as a lubricant in the manufacture of tablets and capsules, and as an enteric coating for gastro-resistant pills and tablets. Dosage as emulsifier: 1-20%.

Lanolin. This component is a mixture of cholesterol and esters of fatty acids chemically related to beeswax. In its commercial quality, if it is good, it does not contain more than 0.25% water, and may contain up to 0.02% of an adequate antioxidant.

It does not become rancid and at the same time it is easily absorbed by human skin, which it softens. Applied on it, it protects it from ry air and maintains body hydration. Its toxicity is practically nil, considering about 15 grams per kilo as a probable lethal dose for humans. One of the characteristics is that it is considered a powerful emollient moisturizer, it contains alcohols that are allergenic for some people.

Glycerin. It is composed of three carbons, eight hydrogens and three oxygens. Its structure has simple links and is tetravalent. Glycerin is a syrupy liquid, colorless and odorless, with a sweet taste to alcohol and insoluble in ether, benzene and chloroform. Of formula C₃H₈0₃ (1,2,3-propanetriol), and relative density of 1.26. It has a boiling point of 290 °C and a melting point of 18 °C. Liquid glycerin is resistant to freezing, but can crystallize at low temperature. It is soluble in water in any proportion, and dissolves in alcohol, but is insoluble in ether and many other organic solvents.

The most frequent use of glycerin is the production of alkyd resins. Other applications are the manufacture of medicines and toiletries, such as toothpaste, as a plasticizing agent for cellophane and as a humidifying agent for tobacco products. Because of its affinity with water and its viscosity, glycerin is used for the ink of sealing pads. It is also used to lubricate the machinery that pumps oil products, due to its resistance to dissolving in petroleum liquids. Due to its high viscosity and absence of toxicity, glycerin is an excellent lubricant for food processing machines. Simple fats and oils are esters of fatty acids and glycerin. Once obtained as a secondary product in the manufacture of the soap after treating the fats and oils with alkali, the crude glycerin is purified by distillation.

Propylenenalicol. It is a colorless liquid, used in the preparation of ointments and creams, which can be incorporated active ingredients, such as corticosteroids. It has also been shown to penetrate through the skin, transporting the incorporated active principles. In addition, it is able to conserve the water contained in the creams. Although it has been pointed out that it may cause allergic sensitization, there is no definitive agreement in this regard.

Triethanolamine. Organic compound derived from ammonia. Viscous, colorless or slightly yellowish hygroscopic liquid, or crystals, with a characteristic odor. It has a molecular mass of 149.2, a boiling point of 335.4 °C, and a melting point of 21.6°C. The disadvantages of this compound are due to its incompatibility with acids, copper salts and heavy metals. Preparations with soaps containing triethanolamine may darken in the presence of light. Additionally it is irritating to the eyes, skin, and respiratory tract. Prolonged or repeated contact may cause skin sensitization.

Citric acid. It is an organic tricarboxylic acid present in most fruits, especially citrus fruits such as lemon and orange. Its chemical formula is C₆H₈O₇. Additionally, it is used in the pharmaceutical industry to achieve effervescence and flavor, and also as blood anticoagulant.

Pharmaceutical formulations in presentation of gel. The gel-type dosage forms are of topical application and can be more or less consistent depending on the amount of gelling agent used. From the physical-chemical point of view, gels are defined as dispersed and uniform systems that consist of two components, a liquid that acts as a dispersing agent and another solid that acts as a structural component and stabilizer of the three-dimensional network.

Pharmaceutical gels are classified, depending on their composition against water, in: a) lipophilic gels (oleo-gels), usually consisting of liquid paraffin with polyethylene or fatty oils gelled with colloidal silica or aluminum or zinc soaps, b hydrophilic gels (hydrogels), constituted by water, glycerol and gelled propylene glycol with the aid of suitable gelling agents such as starch, cellulose derivatives, carbomers and silicates of magnesium and aluminum.

They are also classified, according to the number of phases in which they are constituted, in: a) single-phase gels, where the liquid medium constitutes a single phase or miscible liquids, such as water-alcohol, hydroalcoholic solution, oil; b) biphasic gels, which are constituted by two immiscible liquid phases, forming a transparent structure with semi-solid properties.

In the same way, the gels are classified, according to their viscosity, in: a) fluid gels, b) semi-solid gels, and c) solid gels (formulation of deodorant sticks and solid colognes).

The gelling products can be grouped as follows: i) polymers that give rise to a gel dependent on the pH of the medium, and ii) polymers that give rise to a gel by itself, independent of the pH of the medium. The former give rise to acidic solutions which, when neutralized with the appropriate bases, increase the viscosity and decrease the turbidity of the medium.

The mechanism by which the gel is formed is the following: at low pH values, a small proportion of carboxylic groups of the polymer dissociates, forming a flexible spiral. The addition of a base produces the dissociation of carboxylic groups, ionizing, creating electrostatic repulsion between the charged regions, expanding the molecule, making the system more rigid, gelling it. It goes from a spiral structure to an unrolled or extended, example Carbomer. The seconds do not need to be neutralized for the formation of the gel, they gel by themselves, they form hydrogen bonds between the solvent and the carboxylic groups of the polymer.

In the design of a gel it is essential to select the formulation that presents organoleptic and theological characteristics suitable for its topical administration and ease of use.

This must have the following characteristics:
- Semi-solid or fluid state
- Aspect can be transparent or cloudy
- Presents a structure of continuous type
- Easy application (usually topical)
- pH that is between 4.5 and 8.5.

The gels can be used as lubricants, muscle analgesics, electrocardiography, fluoride dental gels, nasal gels, as excipients for dental treatment, dermal, and intravaginally among others. They increase adhesiveness and thus keep the active ingredient in contact with the skin or mucous membranes (nasal, vaginal, etc.) for a longer period of time. They are used as lubricants and carriers of vaginal spermicides. Another virtue of the gels is that they have a wide range of wetting, therefore their evaporation and the absorption of their active principles can be widely manipulated.

The excipients are those substances that accompany the active principle in the medicine, which are not endowed with pharmacological activity and which are used to improve the appearance, stability, organoleptic properties and bioavailability of the medicinal substances. They are substances that act as solvents, adhesives, disintegrators, dyes, flavorings, preservatives, stabilizers and drug vehicles. They are added to the medicine to improve its stability, its presentation or to facilitate its preparation. Carbopol 940 It is a polymer of acrylic acid, high molecular weight and anionic character. In aqueous solution, hydroalcoholic and with different organic solvents (propylene glycol, glycerin, etc.) and neutralized with alkaline hydroxides or with amines gives a transparent, bright and non-greasy gel, which favors the absorption of the incorporated active principles. The carbopol in aqueous solution has a pH of 2.5 to 3.5, but the stability and viscosity of the gel is maximum at pH between 6 and 11, considerably reducing to pH less than 3 or greater than 12.

In the same way, the gel does not admit percentages greater than 40% in alcohol of 96°. Depending on the percentage of carbomer, the consistency of the gel will increase (0.5% 5%). Solubility: in water it has excellent properties of suspension, thickening and gel formation. Viscosity: 40,000-60,000 centipoises. Organoleptic characteristics: fine white colorless powder. Applications: optimal base for vehiculizing antiseborrhoeic, moisturizing and revitalizing agents. It can be incorporated into emulsions, suspensions and shampoos to increase its viscosity. It also protects the skin against fats and organic solvents.

Triethanolamine. Organic compound derived from ammonia. Viscous, colorless or slightly yellowish hygroscopic liquid, or crystals, with a characteristic odor. It has a molecular mass of 149.2, a boiling point of 335.4 °C, and a melting point of 21.6 °C. The disadvantages of this compound are due to its incompatibility with acids, copper salts and heavy metals. Preparations with soaps containing triethanolamine may darken in the presence of light. Additionally it is irritating to the eyes, skin, and respiratory tract. Prolonged or repeated contact may cause skin sensitization.

Dimethicone. It is a silicone-based polymer frequently used in the manufacture of products for hygiene and personal care, such as creams and lotions for skin, shampoos and soaps. Dimethicone (moisturizes, softens and ensures rapid absorption) forms a protective barrier on the skin to help retain moisture. Due to its oily consistency, it also has emollient properties.

Propylparaben. Also called propylparahydroxybenzoate, nipazaol, protaben, or paseptol. Its chemical formula is HO-C₆H₄-CO₂C₃H₇ and has a molecular mass of 180g/mol, and a melting point of 95-98 °C. It is a white crystalline powder that serves as a reservoir and antimicrobial. It is used mainly in the pharmaceutical, food and cosmetic industries.

The present invention will be described by means of examples, which are only illustrative, and in no way should be understood as limiting the invention. In this sense, any technician in the field could suggest multiple modalities in accordance with the established in the state of the art. Said embodiments also form part of the present invention.

### Example 1

Pharmaceutical formulations in presentation of cream. For the preparation of a batch of 100 g of healing cream to 20% of the active principle we start with the following formula:
i) Stearic acid: 10 grams
ii) Lanolin: 5 grams
iii) Glycerin: 15 grams
iv) Propylene glycol: 10 grams
v) Triethanolamine: 15 grams
vi) Water: 10 grams
vii) Citric acid: 5 grams
viii) Active principle: 20 grams

### Process of preparation of the cream

a. In a 250 ml beaker, triethanolamine, water, and citric acid are slowly mixed.
b. Stearic acid and lanolin are melted in another vessel
c. The glycerin and propylene glycol are also melted.
d. The glycerin/propylene glycol melt is added to the stearic acid/lanolin container.
e. In the mixing of the previous step, the mixture of triethanolamine/water/citric acid is added, and it is stirred at low heat until the formation of a fruit set.
f. We proceed to remove from the fire, and the mixture is stirred until cooled.
g. Finally, the active principle is added.
h. Packaging and labeling

### Quality control of the finished product

The purpose of quality control is to determine if pharmaceutical forms possess the quality characteristics previously established.
a. Determination of the color of the cream. In a clean and dry test tube was filled with the sample halfway, transparency, presence of particles, color was observed.
b. Determination of the odor of the cream. A strip of blotting paper was introduced into the sample and the odor characteristic of the product was determined.
c. Determination of the creaminess of the cream. A small amount of cream was taken and applied on the back of the hand and it was observed if there is presence or absence of fat, what is sought with the untuousness is to know if it is lipophilic or hydrophilic.
d. Determination of the presence of lumps of the cream. A small amount of cream was taken and applied on the back of the hand and observed if there is presence or absence of lumps.
e. Determination of viscosity of the cream. A representative sample of the finished product was taken, the viscometer was introduced and the signal indicated on the viscometer was read.
f. Determination of extensibility of the cream. A small amount of sample was weighed at 25 ° C and pressed between two glass surfaces to which a weight of 100 g is added for one minute. The area originated is the response variable.
g. Determination of the pH of the cream. Measure in the pH meter previously calibrated with buffer solutions, remove the electrode from the buffer wash with distilled water and dry with filter paper. In another container where the sample is, introduce the clean electrode, homogenize and determine the pH.
h. Termoresistance. A sample of cream is applied and left for twelve hours or more at a temperature of 37 °C, there should be no evidence of physical and chemical changes.

### Healing activity test

30 male albino mice of 3 months of age and of 47 g of average weight, were distributed in 3 groups of 10 animals with similar weight. They were kept for four days in individual plastic cages, fed in the morning with 1.5 g 10 g of animal weight.

Subsequently, hair removal was performed in the lower half of the lower back (back of the animal) of each mouse, with warm water and body depilation cream. After 24 hours, when no skin irritation was observed, the animal was placed in a chromatographic tank containing ethyl ether, which serves to anesthetize it to make the wounds of 2 cm in length and 2 mm in depth, with the help of a scalpel Subsequently, 4 hours after the incisions were made, 0.5 g of formulation cream A (positive control, cream containing zinc oxide), formulation B (negative control, cream without active ingredient), or formulation C (cream containing 20% of the active ingredient). The treatments were administered twice a day (morning/afternoon) for the time required.

The healing activity was assessed according to the following parameters:
A) start of healing. The healing activity at the edges of the ulcers was taken into account due to a discrete edema and redness of the affected area, caused by the neovascularization characteristic of the initial healing process.
B) Moderate healing. When the above characteristics were observed in a more extensive area of the edges of the ulcer and the presence of granulation tissue was noted in its interior.
C) Complete healing. When the complete re-epithelialization of the ulcer and its total healing was observed.
D) No healing. When there were no changes of healing in the lesion.
In this sense, the results show that the treatment with the healing cream halves the duration of wound healing.

### Example 2

For the preparation of a 100 g lot of 30% healing gel we start with the following formula:
i) carbopol 940: 2 grams
ii) dimethicone: 2 grams
iii) triethanolamine: 1.8 grams
iv) methylparaben: 0.18 grams
v) Propylparaben: 0.02 grams
vi) water: 74 grams
vii) active ingredient: 20 grams

Process of preparation of the healing gel
a. mix in cold distilled water with carbopol 940, with constant agitation for 30 minutes until formation of a homogeneous gel, which is left at rest with 24 hours in advance to achieve a good hydration.
b. add dimethicone, methylparaben and propylparaben, with agitation.
c. add TEA with slow movement trying not to incorporate air bubbles until it acquires characteristics of gel or pH 6.5.
d. finally the active principle is incorporated.
e. pack in plastic container

### Quality control of the finished product

The quality control of the finished product has the purpose of determining if a pharmaceutical form possesses the quality attributes previously established, these attributes seek to be able to ultimately achieve that the medicine meets the purpose for which it was manufactured safely and effectively.
a. Determination of gel color. In a clean and dry test tube was filled with the sample halfway, transparency, presence of particles, color was observed.
b. Determination of gel odor. A strip of blotting paper was introduced into the sample and the odor characteristic of the product was determined.
c. Determination of the untuosity of the gel. A small amount of gel was taken and applied on the back of the hand and it was observed if there is presence or absence of fat, what is sought with the untuousness is to know if it is lipophilic or hydrophilic.
d. Determination of the presence of gel lumps. A small amount of gel was taken and applied on the back of the hand and observed if there is presence or absence of lumps.
e. Determination of viscosity of the gel. A representative sample of the finished product was taken, the viscometer was introduced and the signal indicated on the viscometer was read.
f. Determination of gel extensibility. A small amount of sample was weighed at 25 °C and pressed between two glass surfaces to which a weight of 100 g is added for one minute. The area originated is the response variable.
g. Determination of the pH of the gel. Measure in the pH meter previously calibrated with buffer solutions, remove the electrode from the buffer wash with distilled water and dry with filter paper. In another container where the sample is, introduce the clean electrode, homogenize and determine the pH.
h. Termoresistance. A gel sample is applied and left for twelve hours or more at a temperature of 37 ° C, no physical or chemical changes should be evidenced.

### Assay of the healing activity

30 male albino mice of 3 months of age and of 47 g of average weight, were distributed in 3 groups of 10 animals with similar weight. They were kept for four days in individual plastic cages, fed in the morning with 1.5 g/10 g of animal weight.

Subsequently, hair removal was performed on the lower third of the back (back of the animal) of each mouse, with warm water and body depilation cream. After 24 hours, when no skin irritation was observed, the animal was placed in a chromatographic tank containing ethyl ether, which serves to anesthetize it to make the wounds of 2 cm in length and 2 mm in depth, with the help of a scalpel

Subsequently, after 4 hours after the incisions were made, 0.5 g of formulation gel A (positive control, cream containing zinc oxide), formulation B (negative control, cream without active ingredient), or formulation C (cream containing 20% of the active principle). The treatments were administered twice a day (morning/afternoon) for the time required.

The healing activity was assessed according to the following parameters.
A) Beginning of healing. The healing activity at the edges of the ulcers was taken into account due to a discrete edema and redness of the affected area, caused by the neovascularization characteristic of the initial healing process.
B) Moderate scarring. When the above characteristics were observed in a more extensive area of the edges of the ulcer and the presence of granulation tissue was noted in its interior.
C) Complete healing. When the complete re-epithelialization of the ulcer and its total healing was observed.
D) No healing. When there were no changes of healing in the lesion.
At the end of the trial the mice were sacrificed by the method of euthanasia with ethyl ether. The extraction of the skin was carried out, to which its macroscopic characteristics were observed, such as color, weight and measurements of length, width and scar length. Subsequently, the skins were placed in containers containing 10% diluted formalin for the corresponding histopathological examination. The histological sections of the skin of each batch were made, then the plates were prepared and finally the microscopic observation was made to determine the cell regeneration. In this sense, the results show that the treatment with the healing gel halves the duration of wound healing.

## Claims

1. A topical pharmaceutical composition useful for the treatment of skin ulcers, injuries and burns, **characterized in that** it comprises: i) indole acetic acid, ii) indole butyric acid, iii) salicylic acid, iv) acetyl benzamide, v) dibenzene carboxylic acid, vi) decanoic acid, vii) dodecanoic acid, Dviii) tetradecanoic acid, Dix) hexadecanoic acid, x) 9-octadecanoic acid, xi) octadecanoic acid methyl ester, xii) 1-decanol, xiii) nonanal, xiv) 13-octadecenal, xv) kinetin, xvi) gibberellic acid, xvii) gibberellins, and an agent selected from the group of a) excipient, b) carrier, and c) pharmaceutically acceptable adjuvant.

2. Topical pharmaceutical composition according to claim 1, **characterized in that** the components indole acetic acid, indoi butyric acid, salicylic acid, acetyl benzamide, dibenzene carboxylic acid, decanoic acid, dodecanoic acid, tetradecanoic acid, hexadecanoic acid, 9-octadecanoic acid, octadecanoic acid methyl ester, 1-decanol, nonanal, 13-octadecenal, kinetin, gibberellic acid, and gibberellins, are in a concentration selected from the range of about 0.001% to about 50%.

3. Topical pharmaceutical composition according to claim 1, **characterized in that** the components indole acetic acid, indole butyric acid, salicylic acid, acetyl benzamide, dibenzene carboxylic acid, decanoic acid, dodecanoic acid, tetradecanoic acid, hexadecanoic acid, 9-octadecanoic acid, octadecanoic acid methyl ester, 1-decanol, nonanal, 13-octadecenal, kinetin, gibberellic acid, and gibberellins, are in a concentration selected from the range of about 1% to about 20%.

4. Topical pharmaceutical composition according to claim 1, **characterized in that** the components indole acetic acid, indoi butyric acid, salicylic acid, acetyl benzamide, dibenzene carboxylic acid, decanoic acid, dodecanoic acid, tetradecanoic acid, hexadecanoic acid, 9-octadecanoic acid, octadecanoic acid methyl ester, 1-decano, nonanal, 13-octadecenai, kinetin, gibberenes, and gibberellins, are in a concentration selected from the range of about 10% to about 20%.

5. Topical pharmaceutical composition according to the preceding claims, **characterized in that** the excipients are selected from the group of stearic acid, lanolin, glycerin, propylene glycol, triethanolamine, citric acid, and combinations thereof.

6. Topical pharmaceutical composition according to the preceding claims, **characterized in that** the excipients are selected from the group of carbopol 940, dimethicone, triethanolamine, methylparaben, propylparaben, and combinations thereof.
